(19) Europäisches Patentamt
European Patent Office
Office européen des brevets

(11) **EP 1 770 353 B1**

(12) **EUROPEAN PATENT SPECIFICATION**

(45) Date of publication and mention
of the grant of the patent:
**02.04.2008 Bulletin 2008/14**

(51) Int Cl.:
***G01B 9/02*** (2006.01) ***A61B 5/00*** (2006.01)

(21) Application number: **06020530.9**

(22) Date of filing: **29.09.2006**

(54) **Optical tomography system**

Vorrichtung zur optischen Tomographie

Système optique de tomographie

(84) Designated Contracting States:
**DE FR GB**

(30) Priority: **30.09.2005 JP 2005289122**

(43) Date of publication of application:
**04.04.2007 Bulletin 2007/14**

(73) Proprietor: **FUJIFILM Corporation**
**Minato-ku**
**Tokyo (JP)**

(72) Inventor: **Toida, Masahiro**
**Ashigarakami-gun,**
**Kanagawa-ken (JP)**

(74) Representative: **Klunker . Schmitt-Nilson . Hirsch**
**Winzererstrasse 106**
**80797 München (DE)**

(56) References cited:
**EP-A2- 1 704 814** **JP-A- 10 332 329**

• YUN S H ET AL: "Pulsed-source and swept-source spectral-domain optical coherence tomography with reduced motion artifacts" OPTICS EXPRESS, OPTICAL SOCIETY OF AMERICA, WASHINGTON, DC,, US, vol. 12, no. 23, November 2004 (2004-11), pages 5614-5624, XP002373645 ISSN: 1094-4087

## Description

BACKGROUND OF THE INVENTION

Field of the Invention

This invention relates to an optical tomography system according to the preamble of claim 1. Such system is used for obtaining an optical tomographic image by measurement of OCT (optical coherence tomography).

Description of the Related Art

As a system for obtaining a tomographic image of an object of measurement in a body cavity, there has been known an ultrasonic tomography system. In addition to such an ultrasonic tomography system, there has been proposed an optical tomography system where an optical tomographic image is obtained on the basis of an interference of light by low coherence light. See, for instance, Japanese Unexamined Patent Publication No. 2003-172690. In the system disclosed in Japanese Unexamined Patent Publication No. 2003-172690, an optical tomographic image is obtained by measuring TD-OCT (time domain OCT) and the measuring light is guided into the body cavity by inserting a probe into the body cavity from the forceps port of an endoscope by way of a forceps channel.

More specifically, low coherence light emitted from a light source is divided into measuring light and reference light and the measuring light is projected onto the object of measurement, while the reflected light from the object of measurement is led to a multiplexing means. The reference light is led to the multiplexing means after its optical path length is changed. By the multiplexing means, the reflected light and the reference light are superposed one on another, and interference light due to the superposition is detected by, for instance, heterodyne detection. In the TD-OCT measurement, a phenomenon that interference light is detected when the optical path of the measuring light conforms to the optical path of the reference light in length is used and the measuring position (the depth of measurement) in the object is changed by changing the optical path length of the reference light.

When measuring the OCT by inserting a probe into a body cavity, the probe is mounted on the system body to be demountable since disinfection, cleaning and the like of the probe after use are necessary. That is, a plurality of probes are prepared for one optical tomography system and the probes are changed by the measurement. However there is an individual difference in the length of the optical fiber due to the manufacturing errors and the like, and the optical path length of the measuring light can change each time the probe is changed. Accordingly, in Japanese Unexamined Patent Publication No. 2003-172690, on the basis of the reflected light from the inner surface of a tube (sheath) covering an optical fiber of the probe, the optical path length of the reference light is adjusted to conform to the optical path length of the measuring light.

Whereas, as a system for rapidly obtaining a tomographic image without changing the optical path length of the reference light such as disclosed in Japanese Unexamined Patent Publication No. 2003-172690, there have been proposed optical tomography systems of obtaining an optical tomographic image by spatially or time dividing the interference light (See, for instance, U.S. Patent No. 5,565,986. Among those, there has been proposed an SS-OCT (swept source OCT) systemwhere interference light is detected by spectrally dividing the interference light in time while the frequency of the light emitted from the light source is swept. In the SS-OCT system, an interferogram interference intensity signal is obtained without changing the optical path length by sweeping the frequency of the laser beam emitted from the light source to cause the reflected light and the reference light to interfere with each other by the use of a Michelson interferometer. Then a tomographic image is generated by carrying out a Fourier analysis on the interferogram signal in the region of an optical frequency.

JP-A-10 332 329 describes a optical coherence tomography system having a light source emitting a light beam whose wavelength is swept. This light beam is divided into a measuring arm and a reference arm at a beam splitter. The reference arm includes a path length adjusting means in the form of a movable mirror. The light reflected at this mirror and the light reflected at the sample in the measuring arm are brought to interference through the beam splitter and a tomographic image is obtained after the detected interference pattern has passed a frequency-analysis in an image obtaining mode. The depth range of the system is set by moving the position of the mirror in the reference arm in a measurement initiating position adjusting mode.

SUMMARY OF THE INVENTION

In the SS-OCT measurement, it is not necessary to conform the optical path length of the measuring light to that of the reference light since information on the reflection in positions in the direction of depth can be obtained by carrying out frequency-analysis However, the wavelength sweeping laser for SS-OCT is actually about 0.1nm in the instant spectral width and about 10mm in coherence length. When the optical path length difference between the measuring light and the reference light is equal to or larger than the coherence length, there is generated no interference. Accordingly, also in the SS-OCT measurement, it is still necessary to adjust the optical path length so that the optical path length of the measuring light conforms to that of the reference light and the measurement initiating position is adjusted to a position in which the object is included in the measurable range.

Further since the measurable range over which a tomographic image is obtainable by the SS-OCT measurement is limited in the direction of depth, it is necessary to adjust the optical path length of the reference light according to the distance between the probe and the object in order to adjust the measurement initiating position so that the object S is positioned in the measurable range. That is, in the SS-OCT measurement, it is necessary to adjust the measurement initiating position so that the object S is positioned in the measurable range in addition to that the optical path length must be adjusted in order to accommodate the individual difference of the probe such as shown in Japanese Unexamined Patent Publication No. 2003-172690.

In the TD-OCT measurement, since the measuring depth is changed by adjusting the optical path length of the reference light, the measurable range can be adjusted by adjusting the optical path length while observing the intensities or the waveforms of the signals obtained by a beat signal measurement or the interferogram measurement of the interference light. However, in the SS-OCT measurement, since the reflection information cannot be obtained unless frequency-analysis such as Fourier-transform is carried out on the detected interference light and even when the position of the object is confirmed to adjust the measurement initiating position, frequency-analysis is required, it takes a long time to adjust the measurement initiating position.

In view of the foregoing observations and description, the primary object of the present invention is to provide an optical tomography system in which the adjustment of the measurement initiating position can be carried out in a short time.

In accordance with the present invention, there is provided an optical tomography system for obtaining a tomographic image of an object to be measured comprising

a light source unit which emits light,

a light dividing means which divides the light emitted from the light source unit into measuring light and reference light,

an optical path length adjusting means which adjusts the optical path length of the measuring light or the reference light divided by the light dividing means,

a multiplexing means which multiplexes the reflected light from the object when the measuring light divided by the light dividing means is projected onto the object and the reference light,

an interference light detecting means which detects interference light of the reflected light and the reference light which have been multiplexed by the multiplexing means,

a tomographic image obtaining means which detects intensities of the interference light in positions in the direction of depth of the object by carrying out frequency-analysis on the interference light detected by the interference light detecting means and obtains a tomographic image of the object, and

a control means which switches between a measurement initiating position adjusting mode in which the position in the direction of depth of the object in which tomographic image signal is to be obtained is adjusted and a tomographic image obtaining mode in which a tomographic image of the object is to be obtained,

wherein the improvement comprises that

the light source unit is provided with a light source which emits light having a predetermined wavelength band and with a wavelength selecting means which selects the wavelength emitted from the light source, and the control means controls the wavelength selecting means and the tomographic image obtaining means so that a laser beam is emitted while the wavelength is swept at a predetermined period and the tomographic image signal is obtained by the tomographic image obtaining means on the basis of the interference light by the laser beam in the image obtaining mode, while controls the wavelength selecting means and the tomographic image obtaining means so that light of a predetermined wavelength is emitted and the tomographic image signal is obtained by the tomographic image obtaining means on the basis of the interference light by the light of a predetermined wavelength in the measurement initiating position adjusting mode.

Further, the control means may have a function, in addition to the function of controlling the light source unit and the image obtaining means according to the mode, of automatically controlling the optical path length adjusting means so that the optical path length difference between the reference light and the measuring light is in an interference signal generating region. The "interference light generating region" means a region where the optical path length difference between the measuring light and the reference light is smaller than the coherence length and interference can occur.

The light source unit may be of any structure so long as it is provided with a light source emitting light having a predetermined wavelength band and with a wavelength selecting means which selects the wavelength emitted from the light source. For example, the light source may be a semiconductor optical amplifier which is connected in loop to an optical fiber, emits spontaneous light, and amplifies the spontaneous light which has been guided along the optical fiber, and the wavelength selecting means may select the spontaneous light to be guided along the optical fiber.

Further, the interference light detecting means may detect an interference light by the light having a predetermined wavelength band as interferogram or a beat signal in the measurement initiating position adjusting mode. When the interference light detecting means detects the interference light as a beat signal, a phase modulation means which gives a frequency difference between the measuring light and the reference light is provided and the control means drives the phase modulation means in the measurement initiating position adjusting mode.

In accordance with the optical tomography system of the present invention, since the light source unit is provided with a light source which emits light having a predetermined wavelength band and with a wavelength selecting means which selects the wavelength emitted from the light source, and the control means controls the wavelength selecting means and the tomographic image obtaining means so that a laser beam is emitted while the wavelength is swept at a predetermined period and the tomographic image signal is obtained by the tomographic image obtaining means on the basis of the interference light by the laser beam in the image obtaining mode, while controls the wavelength selecting means and the tomographic image obtaining means so that light of a predetermined wavelength is emitted and the tomographic image signal is obtained by the tomographic image obtaining means on the basis of the interference light by the light of a predetermined wavelength in the measurement initiating position adjusting mode, the time required for the signal processing to detect the measurement initiating position can be shortened and adjustment of the measurement initiating position can be carried out in a short time when the position in which a tomographic image is to be obtained is adjusted in the measurement initiating position adjusting mode by obtaining the tomographic image and identifying the position of the object by a so-called TD-OCT measurement by the use of the interference light by the ASE light.

Further, when the control means controls the optical path length adjusting means so that the optical path length difference between the reference light and the measuring light is in an interference light generating region in the measurement initiating position adjusting mode, the optical path length adjustment can be automatically carried out, whereby the tomographic image signal can be efficiently obtained and the measurement initiating position can be surely adjusted.

Further, when the light source comprises a semiconductor optical amplifier which is connected in loop to an optical fiber, emits spontaneous light, and amplifies the spontaneous light which has been guided along the optical fiber, and the wavelength selecting means selects the wavelength of the spontaneous light to be guided along the optical fiber, a laser beam where the wavelength is swept at a predetermined period can be emitted in the image obtaining mode, and so-called ASE light can be emitted in the measurement initiating position adjusting mode. Accordingly, a tomographic image at a high resolution can be obtained and at the same time, the measurement initiating position can be accurately adjusted while the light source unit is simple in structure.

When a phase modulation means which gives a frequency difference between the measuring light and the reference light is further provided and the control means drives the phase modulation means in the image obtaining mode, the interference light detecting means can detect the interference light as a beat signal that varies in intensity at the frequency difference, whereby the time required for adjustment of the measurement initiating position can be further shortened.

BRIEF DESCRIPTION OF THE DRAWINGS

Figure 1 is a schematic diagram showing an optical tomography system in accordance with a preferred embodiment of the present invention,
Figure 2 is a view showing fluctuation in wavelength of the laser beam output from the light source unit of Figure 1,
Figure 3 is a schematic diagram showing an optical tomography system in accordance with another embodiment of the present invention, and
Figure 4 is a schematic diagram showing an optical tomography system in accordance with still another embodiment of the present invention.

DESCRIPTION OF THE PREFERRED EMBODIMENTS

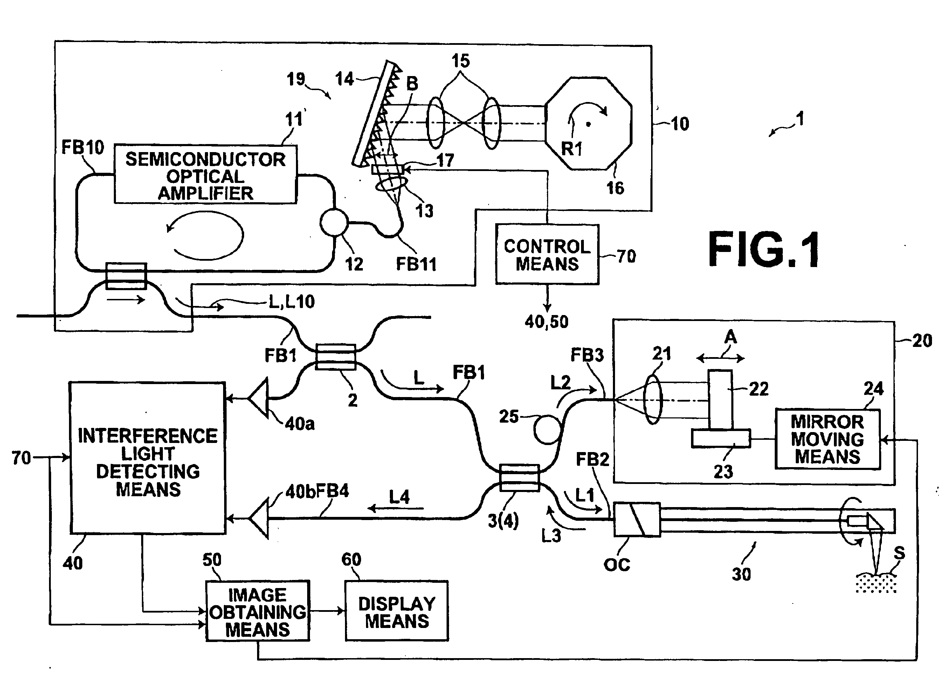

Embodiments of the optical tomography system of the present invention will be described in detail with reference to the drawings, hereinbelow. Figure 1 is a schematic diagram that illustrates an optical tomography system in accordance with a preferred embodiment of the present invention. The optical tomography system 1 of this embodiment is for obtaining a tomographic image of an object of measurement such as a living tissue or a cell in a body cavity by measuring the SS-OCT. The optical tomography system 1 of this embodiment comprises: a light source unit 10 which emits light L; a light dividing means 3 which divides the light L emitted from the light source unit 10 into measuring light L1 and reference light L2; an optical path length adjusting means 20 which adjusts the optical path length of the measuring light L2 divided by the light dividing means; a probe 30 which guides to the object S to be measured the measuring light beam L1 divided by the light dividing means 3; a multiplexing means 4 for multiplexing a reflected light beam L3 from the object S when the measuring light beam L1 is irradiated onto the object S from the probe 30, and the reference light beam L2; an interference light detecting means 40 for detecting interference light beam L4 of the reflected light beam L3 and the reference light beam L2 which have been multiplexed by the multiplexing means 4; and an image obtaining means 50 which detects intensities the interference light beam L4 in positions in the direction of depth of the object by carrying out frequency-analysis on the interference light beam L4 detected by the interference light detecting means and obtains a tomographic image of the object S.

The light source unit 10 is provided with a light source 11 which emits light having a predetermined wavelength band and with a wavelength selecting means 19 which selects the wavelength emitted from the light source 11. The light source 11 is a semiconductor optical amplifier which is connected in loop to an optical fiber FB10, emits spontaneous light, and amplifies the spontaneous light which has been guided along the optical fiber FB10. The light source 11 emits weak spontaneous light to one end of the optical fiber FB10 in response to injection of a drive current and amplifies light input from the other end of the optical fiber FB10.

Whereas, the wavelength selecting means 19 selects the spontaneous light to be guided along the optical fiber FB10 and the spontaneous light is input thereinto through an optical divider 12 linked to the optical fiber FB10. The wavelength selecting means 19 comprises a collimator lens 13, a diffraction grating 14, an optical system 15 and a rotating polygon mirror 16 so that light entering from an optical fiber FB11 travels through the collimator lens 13, the diffraction grating 14 and the optical system 15 and is reflected by the rotating polygon mirror 16. The reflected light is returned to the optical fiber FB11 by way of the optical system 15, the diffraction grating 14 and the collimator lens 13.

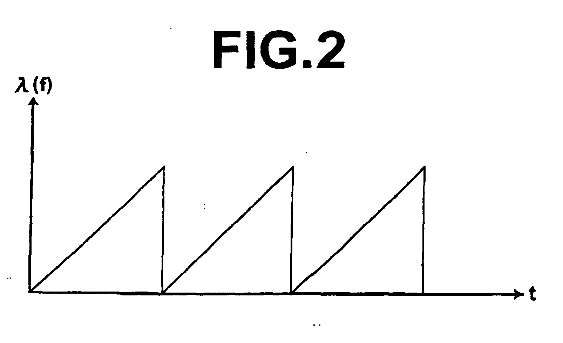

The rotating polygon mirror 16 rotates in the direction indicated by arrow R1, to vary the angle of each reflective surface thereof with respect to the optical axis of the optical system 15. Thereby, only a light beam having a specific frequency, from among the light spectrally split by the diffraction grating 14, is returned to the optical fiber FB11. The frequency of the light beam that reenters the optical fiber FB11 is determined by the angle formed by the optical axis of the optical system 15 and the reflective surface of the rotating polygon mirror 16. Light which comprises a specific frequency band and enters the optical fiber FB11 enters the optical fiber FB10 from the optical divider 12, and as a result, a laser beam L comprising a specific frequency band is emitted to the optical fiber FB1. Accordingly, when the rotating polygon mirror 16 rotates in the direction indicated by arrow R1 at a constant speed, the wavelength of the light beam which reenters the optical fiber FB11 is swept at a period as shown in Figure 2. As a result, a laser beam L which is swept in its wavelength at a period is emitted from the light source unit 10 toward the optical fiber FB1.

The wavelength selecting means 19 is further provided with a mirror 17 which is to be inserted between the collimator lens 13 and the diffraction grating 14 and when the mirror 17 is inserted there, the light emitted from the optical fiber FB11 is returned to the optical fiber FB11 as it is. At this time, the wavelength selecting means 19 selects the wavelength band of the spontaneous light as the wavelength band of the light emitted from the light source unit 10 as a result. This means that the light source unit 10 functions as an ASE light source which emits ASE (amplified spontaneous emission) light L10 by amplifying the spontaneous light. Operation of the mirror 17 is controlled by a control means 70 to be described later.

The light dividing means 3 of Figure 1 comprises, for instance, a 2×2 fiber optic coupler and divides the laser beam L or the ASE light beam L10 led thereto by way of the optical fiber FB1 from the light source unit 10 into the measuring light beam L1 and the reference light beam L2. The light dividing means 3 is optically connected to two optical fibers FB2 and FB3, and the measuring light beam L1 is propagated through the optical fiber FB2 while the reference light beam L2 is propagated through the optical fiber FB3. In Figure 1, the light dividing means 3 also functions as the multiplexing means 4.

The probe 30 is optically connected to the optical fiber FB2 and the measuring light beam L1 is guided to the probe 30 from the optical fiber FB2. The probe 30 is inserted into a body cavity, for instance, through a forceps port by way of a forceps channel and is removably mounted on the optical fiber FB2 by an optical connector OC.

The optical path length adjusting means 20 is disposed on the side of the optical fiber FB3 radiating the reference light beam L2. The optical path length adjusting means 20 changes the optical path length of the reference light beam L2 in order to adjust the tomographic image obtaining area and comprises a collimator lens 21 and a reflecting mirror 22. The reference light beam L2 radiated from the optical fiber FB3 is reflected by the reflecting mirror 22 after passing through the collimator lens 21 and reenters the optical fiber FB3 through the collimator lens 21.

The reflecting mirror 22 is disposed on a movable stage 23 which is moved in the direction of arrow A by a mirror moving means 24. In response to movement of the movable stage 23 in the direction of arrow A, the optical path length of the reference light beam L2 is changed.

The multiplexing means 4 comprises a 2x2 fiber optic coupler, and multiplexes the reference light beam L2 which has been changed in its optical path length and shifted in its frequency by the optical path length adjusting means 20 and the reflected light beam L3 from the object S to emit the multiplexed light beam toward an interference light detecting means 40 by way of an optical fiber FB4.

The interference light detecting means 40 detects interference light beam L4 of the reflected light beam L3 and the reference light beam L2 which have been multiplexed by the multiplexing means 4 and comprises, for instance, a photodiode.

The image obtaining means 50 obtains a tomographic image of the object S by carrying out frequency analysis on the interference light beam L4 detected by the interference light detecting means 40. Then the tomographic image is displayed in a display 60. In the embodiment shown in Figure 1, an optical detector 40a which detects the intensity of the laser light beam L branched from an fiber optic coupler 2 of the optical fiber FB1 and an optical detector 40b which detects the intensity of interference light beam L4 are provided and the interference light detecting means 40 has a function of adjusting the balance of the intensity of the interference light beam L4 on the basis of the output of the optical detector 40a. This function suppresses unevenness in the light intensity by the frequency and permits to obtain a clearer image. The light detecting means 40 has such a spectral sensitivity that it can detect both the wavelength band of the laser beam L and the wavelength band of the ASE light beam L10.

Here, detection of the interference light beam L4 in the interference light detecting means 40 and image generation in the image obtaining means 50 will be described briefly. Note that a detailed description of these two points can be found in M. Takeda, "Optical Frequency Scanning Interference Microscopes", Optical Engineering Contact, Vol. 41, No. 7, pp. 426-432, 2003.

When the measuring light beam L1 is projected onto the object S, the reflected light L3 from each depth of the object S and the reference light L2 interfere with each other with various optical path length difference l. When the light intensity of the interference fringe at this time versus each optical path length difference is assumed to be S(l), the light intensity I(k) detected in the interference light detecting means 40 is expressed by the following formula.

$$I(k) = \int_0^\infty S(l)[l + \cos(kl)]dl \cdots (1)$$

wherein k represents the wave number and l represents the optical path length difference. Formula (1) may be considered to be given as an interferogram of a frequency range having a wave number of $\omega/c$ ($k=\omega/c$) as a variable. Accordingly, a tomographic image is obtained by obtaining in the image obtaining means 50 information on the distance of the object S from the measurement initiating position and information on the intensity of reflection by carrying out frequency analysis by Fourier-transform on the spectral interference fringes detected by the interference light detecting means 40 and determining the intensity S(l) of the interference light beam L4. The tomographic image thus generated is displayed by a display 60.

Operation of the optical tomography system 1 having a structure described above will be described with reference to Figures 1 and 2, hereinbelow. When a tomographic image is to be obtained, the optical path length is first adjusted by moving the movable stage 23 in the direction of the arrow A so that the object S is positioned in the measurable area. The laser beam L is subsequently emitted from the light source unit 10 by sweeping the wavelength at a period and the laser beam L is divided into the measuring light beam L1 and the reference light beam L2 by the dividing means 3. The measuring light beam L1 is led by the optical probe 30 into a body cavity and is projected onto the object S. Then the reflected light beam L3 from the object S and the reference light beam L2 reflected by the reflecting mirror 22 are multiplexed, and the interference light beam L4 of the reflected light beam L3 and the reference light beam L2 is detected by the interference light detecting means 40. A tomographic image is obtained by carrying out frequency analysis on a signal of the detected interference light beam L4 in the image obtaining means 50. In the optical tomography system 1 where a tomographic image is obtained by SS-OCT measurement, image information in the position in the direction of depth is obtained on the basis of the frequency and the intensity of the interference light beam L4, and the movement of the reflecting mirror 22 in the direction of arrow A is employed to adjust the measurement initiating position.

In the case where the measurement initiating position is adjusted by moving the reflecting mirror 22 in the arrow A, steps of first moving the reflecting mirror 22, carrying out detection of the reflected light beam L4 when the reflecting mirror 22 is in the position and signal processing such as frequency-analysis on the detected reflected light beam L4, and thereafter readjusting the position of the reflected mirror 22 is necessary. That is, what kind of interference light beam is detected in the new position of the reflecting mirror cannot be known until the signal processing is carried out, whereby adjustment of the measurement initiating position requires a long time.

Accordingly, in the optical tomography system of Figure 1, there is provided a control means 70 which switches between a measurement initiating position adjusting mode where the position in which a tomographic image is to be obtained is adjusted in the direction of depth of the object S and an image obtaining mode where an image of the object S is obtained so that the system is switched to the image obtaining mode after the position in which a tomographic image is to be obtained is adjusted in the measurement initiating position adjusting mode and a tomographic image is obtained. The control means 70, in the measurement initiating position adjusting mode, inserts the mirror 17 between the collimator lens 13 and the diffraction grating 14 and controls the light source unit 10 to emit only the ASE light L10 and controls the interference light detecting means 40 and the image obtaining means 50 to effect the TD-OCT measurement where the direction of depth of measurement changes in response to movement of the reflecting mirror 22.

Specifically, a phase modulating means 25 such as a piezoelectric element which shifts the frequency of the reference light beam L2 is provided in the optical fiber FB3. In the measurement initiating position adjusting mode, the control means 70 drives the phase modulating means 25 and controls so that the interference light detecting means 40 and the image obtaining means 50 detect the interference light beam L4 by the ASE light beam L10 by heterodyne detection and obtain a tomographic image. Thereby the ASE light beam L10 emitted from the light source unit 10 is divided into the measuring light beam L1 and the reference light beam L2 by the light dividing means 3, and the reflected light beam L3 from the object S is multiplexed with the reference light beam L2 by the multiplexing means 4 to generate the interference light beam L4. In the interference light detecting means 40, a beat signal which repeats strength and weakness at the frequency difference between the reflected light beamL3 and the reference light beamL2 is detected as a signal of the interference light beam L4 when the optical path lengths of the measuring light beam L1 and the reference light beam L2 are equal to each other. The image obtaining means 50 obtains a tomographic image signal from the interference light beam L4. As the optical path length is changed by the optical path length adjusting means 20, the optical path length difference between the measuring light beam and the reference light beam changes and when the optical path lengths of the measuring light beam and the reference beam light come to conform to each other, the beat signal is detected. Accordingly, the measurement initiating position is adjusted by adjusting the position of the reflecting mirror 22 in the optical path length adjusting means 20.

The optical path length adjusting means 20 may be arranged to cause the control means to automatically adjust the optical path length at this time. At this time, the optical path length adjusting means 20 is controlled so that the optical path length difference between the reference light beam L2 and the measuring light beam L1 is in an interference light generating region. The "interference light generating region" means a region where such an interference that the optical path length difference $\Delta l$ between the measuring light beam L1 and the reference light beam L2 is smaller than the coherence length takes place.

After the adjustment of the measurement initiating position, the control means 70 switches from the measurement initiating position adjusting mode to the image obtaining mode and a tomographic image is obtained. At this time, the control means 70 removes the mirror 17 from between the collimator lens 13 and the diffraction grating 14 and controls so that the laser beam L whose wavelength is swept at a constant period is emitted from the light source unit 10. The control means 70 stops the phase modulating means 25. Further, the control means 70 controls the interference light detecting means 40 to detect the interference light beam L4 on which the reflection information in the positions in the direction of depth is superposed. Then the image obtaining means 50 obtains a tomographic image on the basis of the interference light beam L4 detected by the interference light detecting means 40.

By the SS-OCT measurement, where it is not necessary to move the reflecting mirror 22 to obtain a tomographic image, a tomographic image can be obtained at a higher speed than by the TD-OCT measurement. However, the TD-OCT measurement is wider than the SS-OCT measurement in the measurable range. On the other hand, the tomographic image need not be of a high resolution when the measurement initiating position is adjusted. Accordingly, by detecting the object to adjust the optical path length by the TD-OCT measurement in the measurement initiating position adjusting mode, the object S can be easily imaged in a tomographic image and the signal processing can be effected in a short time, whereby the optical path length can be adjusted simply at high speed.

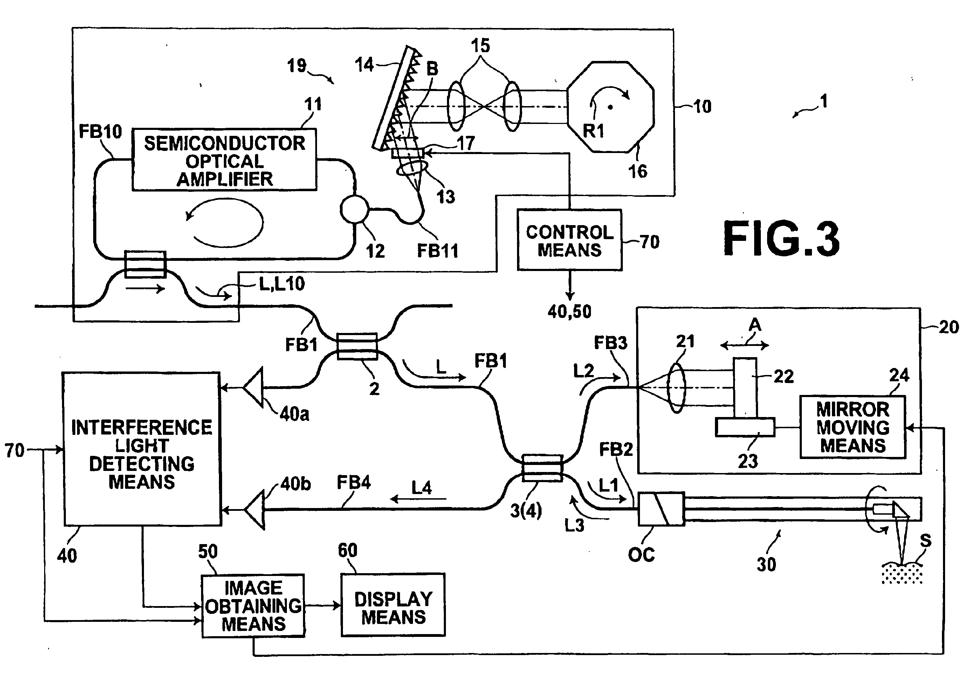

Though, in the measurement initiating position adjusting mode in the above embodiment, the interference light beam L4 by the ASE light L10 is detected as a beat signal, the interference light beam L4 may be detected as an interferogram by not providing the phase modulating means 25 in the optical path of the reference light beam L2 (e.g., the optical fiber FB3) as shown in Figure 3.

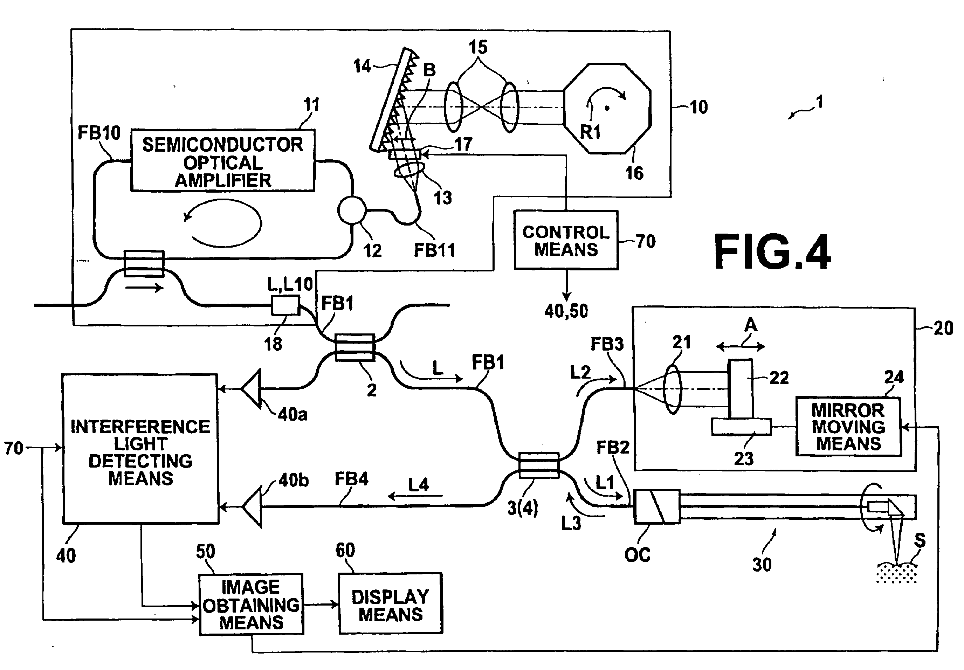

Further, though in the system shown in Figure 1, ASE light beams L10 having the same wavelength band are employed in both the image obtaining mode and the measurement initiating position adjusting mode, a band pass filter 18 may be provided as shown in Figure 4 to narrow the wavelength band of the ASE light beam only in the measurement initiating position adjusting mode.

Further, though the optical path length adjusting means 20 adjusts the optical path length of the reference light beam L2 in Figure 1, the optical path length adjusting means 20 may adjust the optical path length of the measuring light beam L. In this case, the above said optical path length adjusting means 20 is interposed, for instance, in the optical fiber FB2 for guiding the measuring light beam L1 and the mirror in the optical fiber FB3 is fixed.

In accordance with the above embodiments, since the control means 70 controls the wavelength selecting means 19 so that the laser beam L is emitted while the wavelength thereof is swept at a predetermined period in the image obtaining mode and controls the interference light detecting means and the tomographic image obtaining means to detect the interference light beam by the laser beam L and obtain a tomographic image, while controlling the light source unit 10 so that light of a predetermined wavelength band is selected by the wavelength selecting means 19 in the measurement initiating position adjusting mode, and controlling the interference light detecting means and the tomographic image obtaining means to detect the interference light beam by the light beam L10, the time required for the signal processing to detect the measurement initiating position can be shortened and adjustment of the measurement initiating position can be carried out in a short time when the position in which a tomographic image is to be obtained is adjusted in the measurement initiating position adjusting mode by obtaining the tomographic image and identifying the position of the object by a so-called TD-OCT measurement by the use of the interference light beam L4 by the ASE light beam not measuring the distance to the object by the use of the interference light beam L4 by the laser beam L.

## Claims

1. An optical tomography system (1) for obtaining a tomographic image of an object(S) to be measured comprising
   a light source unit (10) which emits light,
   a light dividing means (3) which divides the light emitted from the light source unit (10) into measuring light (L1) and reference light (L2),
   an optical path length adjusting means (20) which adjusts the optical path length of the measuring light (L1) or the reference light (L2) divided by the light dividing means (3),
   a multiplexing means (4) which multiplexes the reflected light (L3) from the object (S) when the measuring light (L1) divided by the light dividing means (3) is projected onto the object (S) and the reference light (L2),
   an interference light detecting means (40) which detects interference light (L4) of the reflected light (L3) and the reference light (L2) which have been multiplexed by the multiplexing means (4),
   a tomographic image obtaining means (50) which detects intensities of the measuring light (L1) in positions in the direction of depth of the object (S) by carrying out frequency-analysis on the interference light (L4) detected by the interference light detecting means (40) and obtains a tomographic image of the object (s),and
   a control means (70) which switches between a measurement initiating position adjusting mode in which the position in the direction of depth of the object (s) in which tomographic image signal is to be obtained is adjusted and a tomographic image obtaining mode in which a tomographic image of the object (s) is to be obtained,
   **characterized in that**
   the light source unit (10) is provided with a light source (11) which emits light having a predetermined wavelength band and with a wavelength selecting means (19) which selects the wavelength emitted from the light source (11), and
   the control means (70) controls the wavelength selecting means (19) and the tomographic image obtaining means (50) so that a laser beam (L) is emitted while the wavelength is swept at a predetermined period and the tomographic image signal is obtained by the tomographic image obtaining means (50) on the basis of the interference light (L4) by the laser beam (L) in the image obtaining mode, while controls the wavelength selecting means (19) and the tomographic image obtaining means (50) so that light of a predetermined wavelength is emitted and the tomographic image signal is obtained by the tomographic image obtaining means on the basis of the interference light (L4) by the light of a predetermined wavelength in the measurement initiating position adjusting mode.

2. An optical tomography system as defined in Claim 1 in which the control means (70) controls the optical path length adjusting means (20) so that the optical path length difference between the reference light (L2) and the measuring light (L1) is in an interference signal generating region.

3. An optical tomography system as defined in Claim 1 or 2 in which the light source (11) is a semiconductor optical amplifier which is connected in loop to an optical fiber, emits spontaneous light, and amplifies the spontaneous light which has been guided along the optical fiber, and the wavelength selecting means (19) selects the wavelength of the spontaneous light to be guided along the optical fiber.

4. An optical tomography system as defined in any one of Claims 1 to 3 further comprising a phase modulation means (25) which gives a frequency difference between the measuring light (L1) and the reference light (L2) in which the control means (70) drives the phase modulation means (25) in the measurement initiating position adjusting mode.

## Patentansprüche

1. Optische Tomographievorrichtung (1) zum Gewinnen eines Tomographiebilds eines zu messenden Objekts (S), umfassend:

   eine Licht emittierende Lichtquelleneinheit (10),
   eine Lichttrenneinrichtung (3), die das von der Lichtquelleneinheit (10) emittierte Licht in Messlicht (L1) und Referenzlicht (L2) trennt,
   eine optische Weglängen-Einstelleinrichtung (20), die die optische Weglänge des Messlichts (L1) oder des Referenzlichts (L2), die von der Lichttrenneinrichtung (3) getrennt wurden, einstellt,
   eine Multiplexeinrichtung (4), die das von dem Objekt (S) beim Projizieren des von der Lichtquelleneinrichtung (3) abgetrennten Messlichts (L1) auf das Objekt (S) von diesem reflektierte Licht (L3) und das Referenzlicht (L2) einer Multiplexbildung unterzieht,
   eine Interferenzlicht-Detektoreinrichtung (40), welche Interferenzlicht (L4) des reflektierten Lichts (L3) und des Referenzlichts (L2), die durch die Multiplexeinrichtung (4) einer Multiplexierung unterzogen wurden, detektiert,
   eine Tomographiebild-Gewinnungseinrichtung (50), die Intensitäten des Messlichts (L1) an Stellen in Tiefenrichtung des Objekts (S) dadurch detektiert, dass sie eine Frequenzanalyse des von der Interferenzlicht-Detektoreinrichtung (40) detektierten Interferenzlichts (L4) durchführt und ein Tomographiebild des Objekts (S) gewinnt, und

eine Steuereinrichtung (70), die umschaltet zwischen einem Messbeginnstellen-Einstellungsmodus, in welchem die Stelle in Tiefenrichtung des Objekts (S), an der ein Tomographiebildsignal gewonnen werden soll, eingestellt wird, und einem Tomographiebild-Gewinnungsmodus, in welchem ein Tomographiebild des Objekts (S) gewonnen werden soll,

**dadurch gekennzeichnet, dass**

die Lichtquelleneinheit (10) mit einer Lichtquelle (11) ausgestattet ist, welche Licht mit einem vorbestimmten Frequenzband emittiert, und mit einer Wellenlängen-Auswahleinrichtung (19) ausgestattet ist, die die von der Lichtquelle (11) emittierte Wellenlänge auswählt, und

die Steuereinrichtung (70) die Wellenlängen-Auswahleinrichtung (19) und die Tomographiebild-Gewinnungseinrichtung (50) derart steuert, dass ein Laserstrahlbündel (L) emittiert wird, während die Wellenlänge mit einer vorbestimmten Periode gewobbelt wird und das Tomographiebildsignal von der Tomographiebild-Gewinnungseinrichtung (50) auf der Grundlage des Interferenzlichts (L4) durch den Laserstrahl (L) in dem Bildgewinnungsmodus erhalten wird, wobei sie die Wellenlängen-Auswahleinrichtung (19) und die Tomographiebild-Gewinnungseinrichtung (50) derart steuert, dass das Licht einer vorbestimmten Wellenlänge emittiert wird und das Tomographiebildsignal von der Tomographiebild-Gewinnungseinrichtung auf der Grundlage des Interferenzlichts (L4) durch das Licht einer vorbestimmten Wellenlänge im Messanfangsstellungs-Einstellmodus gewonnen wird.

2. Vorrichtung nach Anspruch 1, bei der die Steuereinrichtung (70) die optische Weglängen-Einstelleinrichtung (20) derart steuert, dass die optische Weglängendifferenz zwischen dem Referenzlicht (L2) und dem Messlicht (L1) in einer Interferenzsignal-Erzeugungszone liegt.

3. Vorrichtung nach Anspruch 1 oder 2, in der die Lichtquelle (11) ein optischer Halbleiterverstärker ist, der in einer Schleife mit einer optischen Faser gekoppelt ist, spontanes Licht emittiert und das spontane Licht, welches entlang der optischen Faser geleitet wurde, verstärkt, und die Wellenlängen-Auswahleinrichtung (19) die Wellenlänge des spontanen Lichts, das durch die optische Faser zu leiten ist, auswählt.

4. Vorrichtung nach einem der Ansprüche 1 bis 3, weiterhin umfassend eine Phasenmoduliereinrichtung (25), die eine Frequenzdifferenz zwischen dem Messlicht (L1) und dem Referenzlicht (L2) erzeugt, wobei die Steuereinrichtung (70) die Phasenmoduliereinrichtung (25) in dem Messanfangsstellen-Einstellmodus treibt.

**Revendications**

1. Système de tomographie optique (1) destiné à obtenir une image tomographique d'un objet (S) devant être mesuré, comprenant

une unité de source de lumière (10) qui émet de la lumière,

un moyen de division de lumière (3) qui divise la lumière émise depuis l'unité de source de lumière (10) en une lumière de mesure (L1) et une lumière de référence (L2),

un moyen d'ajustement de longueur de trajet optique (20) qui ajuste la longueur de trajet optique de la lumière de mesure (L1) ou de la lumière de référence (L2) divisée par le moyen de division de lumière (3),

un moyen de multiplexage (4) qui multiplexe la lumière réfléchie (L3) à partir de l'objet (S) lorsque la lumière de mesure (L1) divisée par le moyen de division de lumière (3) est projetée sur l'objet (S) et la lumière de référence (L2),

un moyen de détection de lumière d'interférence (40) qui détecte une lumière d'interférence (L4) de la lumière réfléchie (L3) et la lumière de référence (L2) qui ont été multiplexées par le moyen de multiplexage (4),

un moyen d'obtention d'image tomographique (50) qui détecte des intensités de la lumière de mesure (L1) à des positions dans la direction de la profondeur de l'objet (S) en effectuant une analyse fréquentielle sur la lumière d'interférence (L4) détectée par le moyen de détection de lumière d'interférence (40) et obtient une image tomographique de l'objet (s), et

un moyen de commande (70) qui bascule entre un mode d'ajustement de position de début de mesure dans lequel la position dans la direction de la profondeur de l'objet (s) suivant laquelle le signal d'image tomographique doit être obtenu est ajustée et un mode d'obtention d'image tomographique dans lequel une image tomographique de l'objet (s) doit être obtenue,

**caractérisé en ce que**

la source de lumière (10) est dotée d'une source de lumière (11) qui émet de la lumière présentant une bande de longueurs d'onde prédéterminée et d'un moyen de sélection de longueur d'onde (19) qui sélectionne la longueur d'onde émise à partir de la source de lumière (11), et

le moyen de commande (70) commande le moyen de sélection de longueur d'onde (19) et le moyen d'obtention d'image tomographique (50) de sorte qu'un faisceau laser (L) soit émis alors que la longueur d'onde subit un balayage à un intervalle prédéterminé et de sorte que le signal d'image tomographique soit obtenu par le moyen d'obtention d'image tomographique (50) sur la base de la lumière d'interférence (L4) par le faisceau laser (L) dans le mode d'obtention d'image, tout en commandant le moyen de sélection de longueur d'onde (19) et le moyen d'obtention d'image tomographique (50) de sorte que la lumière d'une longueur d'onde prédéterminée soit émise et que le signal d'image tomographique soit obtenu par le moyen d'obtention d'image tomographique sur la base de la lumière d'interférence (L4) par la lumière d'une longueur d'onde prédéterminée dans le mode d'ajustement de position de début de mesure.

2. Système de tomographie optique selon la revendication 1, dans lequel le moyen de commande (70) commande le moyen d'ajustement de longueur de trajet optique (20) de sorte que la différence de longueur de trajet optique entre la lumière de référence (L2) et la lumière de mesure (L1) se trouve dans une région de génération de signal d'interférence.

3. Système de tomographie optique selon la revendication 1 ou 2, dans lequel la source de lumière (11) est un amplificateur optique à semiconducteur qui est connecté en une boucle à une fibre optique, émet une lumière spontanée et amplifie la lumière spontanée qui a été guidée le long de la fibre optique, et le moyen de sélection de longueur d'onde (19) sélectionne la longueur d'onde de la lumière spontanée devant être guidée le long de la fibre optique.

4. Système de tomographie optique selon l'une quelconque des revendications 1 à 3, comprenant en outre un moyen de modulation de phase (25), qui donne une différence de fréquence entre la lumière de mesure (L1) et la lumière de référence (L2), dans lequel le moyen de commande (70) attaque le moyen de modulation de phase (25) dans le mode d'ajustement de position de début de mesure.

FIG.1

# FIG.2

**FIG.3**

FIG.4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003172690 A

- US 5565986 A
- JP 10332329 A

**Non-patent literature cited in the description**

- **M. TAKEDA.** Optical Frequency Scanning Interference Microscopes. *Optical Engineering Contact,* 2003, vol. 41 (7), 426-432